(19) Europäisches Patentamt
European Patent Office
Office européen des brevets

(11) Veröffentlichungsnummer: **0 358 088 B1**

(12) **EUROPÄISCHE PATENTSCHRIFT**

(45) Veröffentlichungstag der Patentschrift: **23.06.93**

(51) Int. Cl.$^5$: **C07C 33/20**, C07C 29/34

(21) Anmeldenummer: **89115892.5**

(22) Anmeldetag: **29.08.89**

(54) **Verfahren zur Herstellung von 3-Aryl-isobutylalkoholen.**

(30) Priorität: **03.09.88 DE 3830019**

(43) Veröffentlichungstag der Anmeldung:
**14.03.90 Patentblatt 90/11**

(45) Bekanntmachung des Hinweises auf die Patenterteilung:
**23.06.93 Patentblatt 93/25**

(84) Benannte Vertragsstaaten:
**BE CH DE ES FR GB IT LI NL**

(56) Entgegenhaltungen:
**EP-A- 0 227 057**
**WO-A-86/07352**

(73) Patentinhaber: **BASF Aktiengesellschaft**
**Carl-Bosch-Strasse 38**
**W-6700 Ludwigshafen(DE)**

(72) Erfinder: **Bott, Kaspar, Dr.**
**Werderstrasse 57**
**W-6800 Mannheim 1(DE)**
Erfinder: **Hoffmann, Herwig, Dr.**
**Knietschstrasse 21**
**W-6710 Frankenthal(DE)**
Erfinder: **Scheidmeir, Walter, Dr.**
**Hardenburgstrasse 41**
**W-6703 Limburgerhof(DE)**

**EP 0 358 088 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Jasmorange, Cyclamenaldehyd und Lysmeral® sind Glieder einer Familie von Aldehyden der Formel

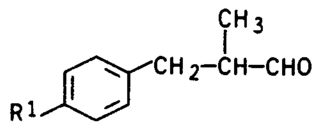

(Jasmorange: $R^1$ = $-CH_3$; Cyclamenaldehyd: $R^1$ = $-CH(CH_3)_2$ Lysmeral®: $R^1$ = $-C(CH_3)_3$), die als Duftstoffe in der Parfüm-Industrie besonders begehrt sind (vgl. Ullmann's Encyclopedia of Ind. Chem., Vol. A1, Seite 343).

Weiterhin sind die genannten Aldehyde, aber auch die entsprechenden Alkohole bzw. Halogenverbindungen der Formel

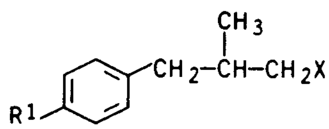

(X = -OH oder Halogen)
als technisch sehr interessante Zwischenprodukte auf dem Pflanzenschutzgebiet anzusehen, weil durch Umsetzen dieser Verbindungen mit Morpholinderivaten hochwirksame Fungizide hergestellt werden können (vgl. Angewandte Chemie 92 (1980), Seiten 176 bis 181, bes. Seite 179).

Eine allgemein anwendbare und technisch bisher bevorzugt ausgeübte Synthese der oben genannten Verbindungen geht von den entsprechend substituierten Benzaldehyden aus, die in einer ersten Stufe durch Aldolkondensation mit Propionaldehyd zu den entsprechenden 3-Aryl-2-methyl-2-propenalen umgesetzt werden. Letztere werden anschließend mit katalytisch angeregtem Wasserstoff zu den entsprechenden gesättigten Aldehyden oder zu den entsprechenden gesättigten Alkoholen hydriert. Somit nehmen die alkylsubstituierten Benzaldehyde eine Schlüsselstellung für den Aufbau des Kohlenstoffgerüstes der obengenannten beliebten Riechstoffe sowie der Pflanzenschutzmittelzwischenprodukte ein.

Die substituierten Benzaldehyde werden aber in der Technik im allgemeinen durch Oxydation der entsprechenden Akylbenzole - sei es elektrochemisch, mit Sauerstoff oder über eine Halogenierung - produziert, wobei die Oxidationsstufe des betreffenden Benzylalkohols in irgendeiner Form durchlaufen werden muß. Aus diesem Grund sollte es möglich sein, die Synthese der 3-Aryl-2-methyl-propanale der entsprechenden Alkohole und Halogenide wirtschaftlicher zu gestalten, wenn man das zum Aufbau der genannten Verbindungen angewendete Syntheseprinzip auf die Benzylalkohole als Ausgangsstoffe übertragen könnte.

Es war daher die Aufgabe der Erfindung, einen einfacheren und daher wirtschaftlicheren Weg zur Herstellung alkylsubstituierter Phenylisobutylalkohole bzw. -isobutyraldehyde ausgehend von entsprechend substituierten Benzylalkoholen zu entwickeln.

Es wurde nun überraschenderweise gefunden, daß man die 3-Aryl-isobutylalkohole der allgemeinen Formel I

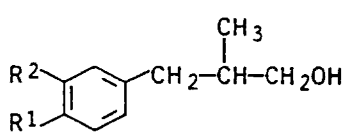

(I),

in der

$R^1$ und $R^2$ für Wasserstoff oder einen Alkyl- bzw. Cycloalkylrest mit bis zu 8 C-Atomen, vorzugsweise einen Alkylrest mit 1 bis 4 C-Atomen stehen, in sehr guten Ausbeuten bei technisch akzeptablen Umsätzen erhält,

wenn man die entsprechenden Arylcarbinole der allgemeinen Formel II

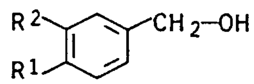

(II),

in Gegenwart katalytischer Mengen, d.h. etwa in Mengen von 0,5 bis 2 Gew.%, bezogen auf das Reaktionsgemisch, eines Alkalihydroxids oder eines Alkalialkoholats, vorzugsweise NaOH oder KOH, insbesondere KOH, bei 250 bis 350 °C in einem geschlossenen Reaktionsgefäß mit n-Propanol umsetzt, wobei auf die Anwesenheit nennenswerter Mengen eines Dehydrierungskatalysators und das Abdestillieren des während der Reaktion gebildeten Wassers verzichtet wird.

Es war überraschend, daß die Arylisobutanole der Formel I nach dem oben beschriebenen Verfahren in ausgezeichneten Ausbeuten bei technisch akzeptablen Umsätzen erhalten werden können, auch wenn man keinen der Ausgangsstoffe im Überschuß einsetzt, d.h. im Molverhältnis von etwa 1:1 und in Abwesenheit nennenswerter Mengen eines Dehydrierungskatalysators und/oder eines sonstigen Zusatzkatalysators arbeitet und auf das kontinuierliche Entfernen des während der Reaktion gebildeten Reaktionswassers verzichtet.

Die durch Basen katalysierte Kondensationsreaktion von zwei niederen Alkoholen zu höheren Alkoholen ist zwar eine schon lange bekannte Reaktion (Markownikoff-Guerbet-Reaktion; vgl. Houben-Weyl), Methoden der Organischen Chemie, 4. Auflage, Band VI/1b, Georg-Thieme-Verlag, Stuttgart, New York, 1984, Seiten 645 bis 652, bes. Seite 650), jedoch ließen die dabei erzielten Ausbeuten im allgemeinen sehr zu wünschen übrig. Sie konnte im allgemeinen nur wirtschaftlich betrieben werden, wenn man das entstehende Reaktionswasser kontinuierlich aus dem Reaktionsgefäß eliminierte (vgl. loc. cit. S. 647), was nach dem Stand der Technik besonders wichtig ist bei Verwendung von Alkalihydroxiden als basischem Kondensationsmittel (vgl. loc. cit. S. 648). Hierdurch wird die Reaktionsführung recht aufwendig.

Des weiteren wurden zur Beschleunigung der Umsetzung und Steigerung der Selektivität Zusätze von Dehydrierungskatalysatoren, wie aktives Nickel, Kupfer oder Kupferbromid und gar eine Modifizierung der Dehydrierungskatalysatoren durch Phosphane und Arsane und einen Zusatz von WasserstoffAkzeptoren, wie Bleisalzen, beschrieben (vgl. loc. cit. Seite 648). Nachteilig hieran ist, daß die Abtrennung der teilweise gesundheitsgefährdenden Verbindungen die Aufarbeitung des Reaktionsgemisches erschwert.

Neben den in der Literatur hauptsächlich beschriebenen Dimerisierungen von niederen Alkoholen (vgl. DE 1 443 666; US 2 457 866 und US 3 119 880) sind auch Cokondensationen zweier primärer Alkohole bekannt. Gemäß loc. cit., Seite 650 heißt es zwar, daß für Cokondensationen Benzylalkohol gut geeignet ist, jedoch wird gemäß der dort angegebenen Arbeitsvorschrift das 3-Phenyl-isobutanol (dort 2-Benzyl-propanol genannt) durch Umsetzen von Benzylalkohol mit Propanol in Gegenwart von Natrium und Kupferbronze im Autoklaven nur in einer technisch absolut undiskutablen Ausbeute von 32 % der Theorie erhalten. Auch die auf den Seiten 651 und 652 von loc. cit. tabellarisch angegebenen Ausbeuten für durch Kaliumalkoholat katalysierte Cokondensationen von Alkoholen in Gegenwart von aktivem Nickel bzw. von Kupferbronze in der Größenordnung von 8 bis maximal 62 % ließen nicht erwarten, daß die begehrten 3-Aryl-isobutanole der Formel I durch einfaches Umsetzen der Alkohole in Gegenwart katalytischer Mengen eines Akalihydroxids ohne weitere die Umsetzung verteuernde und die Aufarbeitung erschwerende Maßnahmen in außerordentlich guten Ausbeuten erhalten werden können.

Dies gilt besonders, da gemäß loc. cit. die höheren Ausbeuten, wie 62 %, nur dadurch erreicht werden konnten, daß eine Wasser-Abscheidung aus dem Reaktionsgemisch erfolgte, wie in der Tabelle auf Seite 651 angegeben ist.

Außerdem war aus WO-A-8 607 352 bekannt, daß man Benzylalkohol mit höheren Alkanolen in Gegenwart von alkalisch reagierenden Alkalimetallverbindungen zu den entsprechenden 2-Benzyl-alkanolen mit Ausbeuten von 64 bzw. 74 % erhalten kann, jedoch sind im Gegensatz zum Verfahren der vorliegenden Erfindung die hierbei eingesetzten $C_8$- bis $C_{25}$-Alkanole sehr hoch siedend und nicht mit Wasser mischbar, wodurch sich das bei der Reaktion bildende Wasser entweder als separate Phase abscheiden oder mit überschüssigem Benzylalkohol azeotrop abdestilliert werden kann. Die oben angegebenen Ausbeuten konnten nur bei Abdestillieren des Reaktionswassers und zusätzliche Anwesenheit eines Dehydrierungskatalysators erzielt werden.

Weiterhin war aus EP 227 057 die Umsetzung gemäß folgender Reaktionsgleichung bekannt:

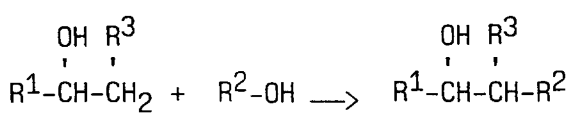

,

worin $R^1$ für einen aromatischen oder heterocyclischen Rest, $R^2$ für einen Alkylrest mit bis zu 20 C-Atomen und $R^3$ für H, $-CH_3$ oder $-C_2H_5$ stehen. Wie aus der Reaktionsgleichung zu entnehmen ist, reagieren die hierbei eingesetzten sekundären aromatischen Alkohole ganz anders als die gemäß dem vorliegenden Verfahren verwendeten primären aromatischen Alkohole, da die OH-Gruppe der sekundären Alkohole bei der Umsetzung erhalten bleibt. Gemäß dem Verfahren der EP 227 057 werden zwar recht gute Selektivitäten an dem gewünschten höheren Alkohol erzielt, technisch akzeptable Umsätze erhält man aber nur, wenn man mit Alkoholen arbeitet, die nicht mit Wasser mischbar sind und sich daher das bei der Reaktion gebildete Wasser durch Phasentrennung aus dem organischen Reaktionsgemisch abtrennen läßt.

Als Arylcarbinole der allgemeinen Formel II sind insbesondere solche geeignet, in denen $R^1$ für einen Alkylrest mit 1 bis 4 C-Atomen und $R^2$ für H stehen. Genannt seien insbesondere p-Methyl-benzylalkohol, p-Isopropylbenzylalkohol und p-tert.-Butyl-benzylalkohol.

Als basische Katalysatoren sind Alkalihydroxide, vorzugsweise NaOH und KOH, insbesondere KOH, oder auch Alkalialkoholate geeignet.

Die basischen Katalysatoren verwendet man in Mengen von 0,5 bis 2, vorzugsweise 0,6 bis 1,6 Gew.%, bezogen auf das Reaktionsgemisch, entsprechend etwa 1 bis 3, vorzugsweise etwa 1,5 bis 2,5 Mol.%, bezogen auf die Alkohole.

Die Reaktionstemperaturen betragen 250 bis 350 °C, vorzugsweise 260 bis 290 °C; die Reaktionszeiten etwa 5 bis 12 Stunden.

Man arbeitet im geschlossenen Reaktionsgefäß, weil die Dampfdrücke der Komponenten in der Reaktionsmischung zusammen über dem Normaldruck liegen.

Mit Hilfe des erfindungsgemäßen Verfahrens können die als Vorprodukte für die begehrten Riechstoffe Jasmorange, Cyclamenaldehyd und Lysmeral® (BASF) bzw. als Zwischenprodukte für hochwirksame Fungizide bedeutsamen 3-Arylisobutanole der allgemeinen Formel I auf einfache und wirtschaftliche Weise in überraschend guten Ausbeuten erhalten werden. Die Alkohole der Formel I können auf einfache Weise durch Oxidation mit Sauerstoff oder durch einfache Dehydrierung in die Riechstoffe selbst überführt werden.

Beispiele

Beispiel 1

In einem 5-l-Rührautoklaven (Material RA2) wurde eine Mischung aus 2484 g (23 mol) Benzylalkohol, 1380 g (23 mol) n-Propanol und 38 g (0,68 mol) Kaliumhydroxid 10 Stunden (h) auf 270 °C erhitzt. Bei der destillativen Aufarbeitung des Reaktoraustrages in einer Füllkörperkolonne (30 cm Füllhöhe, Füllkörper: RA2-Netze) wurden neben unumgesetztem n-Propanol und 2-Methyl-pentanol (gebildet durch Eigenkondensation von n-Propanol) als Hauptbestandteile 1339 g nicht umgesetzter Benzylalkohol und 1445 g 3-Phenyl-isobutanol (Siedepunkt 84 °C/0,3 mbar) isoliert. Die Ausbeute an 3-Phenyl-isobutanol betrug 91 % d. Theorie, bezogen auf umgesetzten Benzylalkohol bei einem Umsatz von etwa 46 %.

Beispiele 2 bis 4

In einem Rührautoklaven wurde jeweils eine Mischung aus den aus der folgenden Tabelle ersichtlichen Mengen an den Alkoholen der Formel II und n-Propanol sowie der dort angegebenen Menge an Kaliumhydroxid 15 h auf 270 °C erhitzt. Der Autoklavenaustrag wurde analog Beispiel 1 destillativ aufgearbeitet. Die bei dieser Umsetzung erzielten Umsätze sowie die auf den umgesetzten Benzylalkohol der Formel II bezogenen Ausbeuten an Alkoholen der allgemeinen Formel I sind in der Tabelle angegeben.

Tabelle

| Beispiel | Benzylalkohole (mol) | | n-Propanol (mol) | Katalysator (g) | Isobutylalkohole der Formel I | Umsatz [%] | Selektivität [% d.Th.] |
|---|---|---|---|---|---|---|---|
| 2 | p-Methyl- | ( 1,0) | (1,0) | KOH (1,8) | 3-(p-Methyl-phenyl)- | 42 | 93 |
| 3 | p-Isopropyl- | ( 0,67) | (1,0) | KOH (1,6) | 3-(p-Isopropyl-phenyl)- | 46 | 92 |
| 4 | p-tert.-Butyl- | (14) | (14) | KOH (31,5) | 3-(p-tert.-Butyl-phenyl)- | 44 | 92 |

## Patentansprüche

**1.** Verfahren zur Herstellung von 3-Aryl-isobutylalkoholen der allgemeinen Formel I

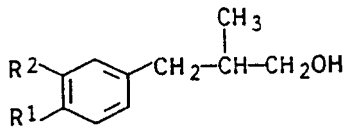 (I),

in der

$R^1$ und $R^2$ für Wasserstoff oder einen Alkyl- bzw. Cycloalkylrest mit bis zu 8 C-Atomen, vorzugsweise einen Alkylrest mit 1 bis 4 C-Atomen stehen,

dadurch gekennzeichnet, daß man Arylcarbinole der allgemeinen Formel II

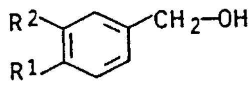 (II),

in Gegenwart katalytischer Mengen eines Alkalihydroxids oder eines Alkalialkoholats bei 250 bis 350 °C in einem geschlossenen Reaktionsgefäß mit n-Propanol umsetzt, wobei auf die Anwesenheit nennenswerter Mengen eines Dehydrierungskatalysators und das Abdestillieren des während der Reaktion gebildeten Wassers verzichtet wird.

**2.** Verfahren gemäß Anspruch 1, dadurch gekennzeichnet, daß man das Arylcarbinol der Formel II in Gegenwart von 0,5 bis 2 Gew.% an NaOH oder KOH, bezogen auf das Reaktionsgemisch, mit dem n-Propanol umsetzt.

**3.** Verfahren gemäß Anspruch 1, dadurch gekennzeichnet, daß man das Arylcarbinol der Formel II mit n-Propanol im Molverhältnis von etwa 1:1 umsetzt.

**4.** Verfahren gemäß Anspruch 1, dadurch gekennzeichnet, daß man ein Arylcarbinol der allgemeinen Formel II

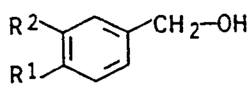 (II),

in der $R^1$ für einen Methylrest, einen Isopropylrest oder einen tert.-Butylrest steht und $R^2$ Wasserstoff bedeutet, mit dem n-Propanol umsetzt.

## Claims

**1.** A process for the preparation of a 3-arylisobutyl alcohol of the formula I

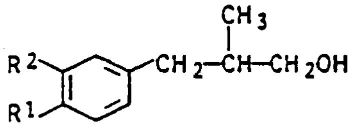 (I)

where $R^1$ and $R^2$ are each hydrogen or an alkyl or cycloalkyl radical of not more than 8 carbon atoms, preferably alkyl of 1 to 4 carbon atoms, wherein an arylcarbinol of the formula II

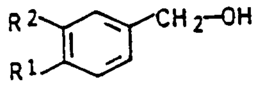

(II)

is reacted with n-propanol in the presence of a catalytic amount of an alkali metal hydroxide or of an alkali metal alcoholate at from 250 to 350 °C in a closed reaction vessel, the presence of a significant amount of a dehydrogenation catalyst and distilling off the water formed during the reaction being dispensed with.

**2.** A process as claimed in claim 1, wherein the arylcarbinol of the formula II is reacted with the n-propanol in the presence of from 0.5 to 2% by weight, based on the reaction mixture, of NaOH or KOH.

**3.** A process as claimed in claim 1, wherein the arylcarbinol of the formula II is reacted with n-propanol in a molar ratio of about 1 : 1.

**4.** A process as claimed in claim 1, wherein an arylcarbinol of the formula II

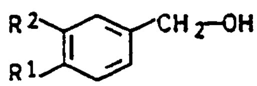

(II)

where $R^1$ is methyl, isopropyl or tert-butyl and $R^2$ is hydrogen, is reacted with the n-propanol.

**Revendications**

**1.** Procédé de préparation d'alcools 3-arylisobutyliques de formule générale I

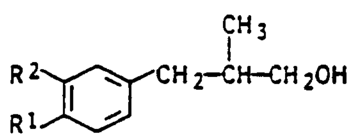

(I),

dans laquelle

$R^1$ et $R^2$ sont mis pour des atomes d'hydrogène ou pour des restes alkyle ou cycloalkyle renfermant jusqu'à 8 atomes de carbone, de préférence pour des restes alkyle à 1-4 atomes de carbone,

caractérisé qu'on fait réagir des arylcarbinols de formule générale II

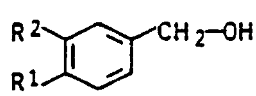

(II),

avec du n-propanol en présence de quantités catalytiques d'un hydroxyde alcalin ou d'un alcoolate alcalin, à une température de 250 à 350 °C dans un réacteur fermé, en évitant ainsi la présence de quantités appréciables d'un catalyseur de déshydrogénation et l'élimination par distillation de l'eau formée au cours de la réaction.

**2.** Procédé selon la revendication 1, caractérisé en ce qu'on fait réagir l'arylcarbinol de formule II avec du n-propanol en présence de 0,5 à 2% en poids de NaOH ou de KOH par rapport au mélange réactionnel.

**3.** Procédé selon la revendication 1, caractérisé en ce qu'on fait réagir l'arylcarbinol de formule II avec du n-propanol dans un rapport molaire d'environ 1:1.

4. Procédé selon la revendication 1, caractérisé en ce qu'on fait réagir avec du n-propanol un arylcarbinol de formule générale II

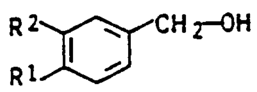

(II),

dans laquelle $R^1$ est mis pour un reste méthyle, un reste isopropyle ou un reste tert.-butyle et $R^2$ représente un atome d'hydrogène.